# EUROPEAN PATENT APPLICATION

(11) **EP 4 567 099 A1**
(43) Date of publication of application: **11.06.2025**
(21) Application number: 23849490.0
(22) Date of filing: 03.08.2023
(51) Int. Cl.: C12N 1/20, A61K 35/74, A61P 17/00

(54) **CUTIBACTERIUM ACNES STRAIN, AND USE, COMPOSITION AND DRUG THEREOF**

(30) Priority: 04.08.2022 CN 202210935678
(71) Applicant: Shenzhen 01 Life Science and Technology Co., Ltd., Shenzhen, Guangdong 518100 (CN)
(72) Inventor: LI, Wei, Shanghai 200040 (CN); QIU, Zhuoqiong, Shanghai 200040 (CN); YU, Tianze, Shanghai 200040 (CN); XU, Xiaoqiang, Shenzhen, Guangdong 518100 (CN); WANG, Xiaokai, Shenzhen, Guangdong 518100 (CN)
(74) Representative: Grünecker Patent- und Rechtsanwälte PartG mbB
(86) International application number: PCT/CN2023/110917
(87) International publication number: WO 2024/027786

(57) **Abstract**

The present invention provides a *Cutibacterium acnes* AD3 strain, GDMCC No: 62625. The strain features high propionic acid yields, and can be used for preparing a product for treating an inflammation-related skin disease and improving skin conditions.

## Description

The present application claims priority to Chinese Patent Application No. 202210935678.3, titled "CUTIBACTERIUM ACNES STRAIN, AND USE, COMPOSITION AND DRUG THEREOF" filed with the China National Intellectual Property Administration on August 4, 2022, the entirety of which is incorporated herein by reference.

### TECHNICAL FIELD

The present invention relates to the field of biomedical technology, and specifically to a *Cutibacterium acnes,* and a use, composition, and medicament thereof.

### BACKGROUND

Atopic dermatitis (AD) is a common chronic, recurrent, and inflammatory skin disease characterized by intense pruritus, eczematous dermatitis (features include erythema, infiltration/papules, exudation with crusting, epidermal shedding, and lichenification), skin dryness, and a family history of allergies. It is also associated with an increased risk of various comorbidities, including food allergies, asthma, allergic rhinitis, and mental health disorders, severely impacting the physical and mental well-being of patients. In recent years, the prevalence of atopic dermatitis has been steadily increasing, affecting individuals of nearly all ages and ethnicities. Its recurrent disease course and intractable skin pruritus impose significant socioeconomic and psychological burdens on patients and their families, making it one of the leading causes of the global dermatological disease burden. The etiology of atopic dermatitis remains unclear to date. Studies suggest that it may be associated with skin barrier dysfunction, immune bias in the microenvironment, microbial imbalance on the skin, genetic factors, and environmental factors. Currently, the treatment of atopic dermatitis primarily focuses on conventional topical therapies (such as topical corticosteroids, calcineurin inhibitors, antibiotics, antipruritics, and emollients), oral medications (including antihistamines, systemic corticosteroids, and immunosuppressants), and biologic therapies, etc.

In most cases, topical corticosteroids (TCS) are the most commonly prescribed type of drugs in clinical practice. However, due to risks such as skin atrophy, pigmentation abnormalities, acneiform eruptions, and systemic absorption-related effects (e.g., hypothalamic-pituitary axis suppression and Cushing's syndrome), the long-term use of TCS is not recommended. Topical calcineurin inhibitors (TCIs) are generally effective and safe for short-term treatment. However, concerns regarding the risk of skin malignancies and an increased risk of lymphoma have led regulatory authorities to mandate the inclusion of warnings related to long-term safety in the prescribing information for tacrolimus and pimecrolimus. Use of any topical treatment, either repeatedly over an extended period or applied across large areas of the body, can lead to decreased patient compliance. Oral immunosuppressants and systemic corticosteroids can effectively treat AD, but their use is often accompanied by severe toxicity and side effects, restricting their application to short-term or intermittent therapy. Systemic corticosteroid therapy is associated with side effects such as diabetes, hypertension, and osteoporosis, along with the risk of rebound flare-ups after discontinuation. Biologic therapies, such as anti-tumor necrosis factor-alpha (TNF-α) agents (infliximab and etanercept), anti-IgE (omalizumab), anti-IL-5 (mepolizumab), and anti-CD11a (efalizumab), have generally shown ineffectiveness in clinical trials. Biologics targeting IL-4Rα (dupilumab) have demonstrated better therapeutic effects, but approximately one-third of patients still exhibit poor responses. Small-molecule targeted drugs, such as JAK inhibitors (upadacitinib and abrocitinib), have also shown promising therapeutic efficacy; however, their mechanism of blocking T-helper cell signaling may lead to adverse immunosuppressive effects. Thus, there remains a significant unmet medical need for effective therapeutic options for atopic dermatitis (AD).

Similar to the therapeutic effects of oral probiotics on atopic dermatitis (AD), research on topical applications of skin microbiota for AD treatment has also made certain advancements. For example, animal studies have demonstrated that lysates of non-pathogenic *Vitreoscilla filiformis* can suppress skin inflammation in mice. Small-scale clinical trials have shown that topical application of *Rhodococcus* species can alleviate symptoms in AD patients. Additionally, a Phase I randomized double-blind clinical trial indicated that *Staphylococcus hominis* has therapeutic potential for AD patients. These studies suggest that the topical application of skin microbiota represents an important therapeutic strategy for atopic dermatitis.

Compared to healthy individuals, AD patients exhibit reduced bacterial diversity in their skin microbiota, particularly in affected areas. Moreover, the relative abundance of *Staphylococcus aureus* and *Staphylococcus epidermidis* is increased.

### CONTENT OF THE INVENTION

In view of the foregoing, the present invention aims to address the technical problem of providing a *Cutibacterium acnes,* and a use, composition, and medicament thereof. The *Cutibacterium acnes* provided by the present invention demonstrates high yield of propionic acid and is applicable for preparing a skin-improving product for at least one of treating skin-related inflammation, promoting the balance and homeostasis of skin symbiotic microbiota, enhancing skin barrier function, repairing skin damage, and suppressing pruritus.

The present invention provides a *Cutibacterium acnes* with a deposit accession number as GDMCC No: 62625 and named *Cutibacterium acnes* AD3, with the Latin designation *Cutibacterium acnes* AD3.

The *Cutibacterium acnes* as mentioned above, along with at least one of a cellular component thereof, a metabolite thereof, a derivative of the metabolite thereof, or a secretion thereof, falls within the protection scope of the present invention. It is understood that the cellular component includes at least one of a cell, a culture media containing the cell, and various chemical constituents of a cell; the metabolite includes at least one of intermediate metabolites and final metabolites in metabolism; the secretion includes at least one of nucleic acids, enzymes, antibodies, exosomes, and hormones.

The *Cutibacterium acnes* described herein was extracted from the perilesional skin of atopic dermatitis patients and obtained through screening, cultivation, and purification, which exhibits high propionic acid production. In certain examples of the present invention, the propionic acid produced by every 100,000 CFU of the *Cutibacterium acnes* is 200 µg to 1,000 µg after 24 hours of anaerobic cultivation.

The *Cutibacterium acnes* of the present invention can inhibit the expression of inflammatory factors through metabolic product, propionic acid, thereby achieving an effect of treating inflammation. For example, it inhibits the expression of inflammatory factors in keratinocytes via metabolic product, propionic acid, achieving the effect of treating inflammatory skin-related diseases. In certain examples of the present invention, the present invention provides a use of at least one of the *Cutibacterium acnes,* the cellular component thereof, the metabolite thereof, the derivative of the metabolite thereof, or the secretion thereof as mentioned above in the preparation of an inhibitor of the expression of an inflammatory factor; the inflammatory factor includes at least one of IL-33, IL-1, IL-4, IL-5, IL-6, IL-8, IL-12, IL-13, IL-18, IL-25, IL-31, TSLP, IL-17, IL-22, IL-23, TNF-α, and IFN-γ, etc. In certain examples of the present invention, the present invention provides a use of at least one of the *Cutibacterium acnes,* the cellular component thereof, the metabolite thereof, the derivative of the metabolite thereof, or the secretion thereof as mentioned above in the preparation of a skin-improving product for treating an inflammatory skin-related disease. In one example, the inflammatory skin-related disease includes at least one of atopic dermatitis, eczema, rosacea, seborrheic dermatitis, contact dermatitis, psoriasis, acne, vitiligo, urticaria, alopecia areata, androgenetic alopecia; autoimmune skin diseases such as lupus erythematosus, scleroderma, and dermatomyositis, etc.; bullous skin diseases such as pemphigus and bullous pemphigoid, etc.; infectious skin diseases such as fungal infectious skin diseases, bacterial infectious skin diseases, and viral infectious skin diseases; and skin tumors such as benign skin tumors including seborrheic keratosis and actinic keratosis, and malignant skin tumors including basal cell carcinoma and squamous cell carcinoma.

The *Cutibacterium acnes* of the present invention can inhibit the proliferation of *Staphylococcus aureus,* thereby achieving the effect of treating a related disease caused by *Staphylococcus aureus,* and stabilizing the balance of the skin symbiotic microbiota. In certain examples of the present invention, the present invention provides a use of at least one of the *Cutibacterium acnes,* the cellular component thereof, the metabolite thereof, the derivative of the metabolite thereof, or the secretion thereof as mentioned above in the preparation of an inhibitor of *Staphylococcus aureus.* In certain examples of the present invention, the present invention provides a use of at least one of the *Cutibacterium acnes,* the cellular component thereof, the metabolite thereof, the derivative of the metabolite thereof, or the secretion thereof as mentioned above in the preparation of a skin-improving product for promoting the balance and homeostasis of skin symbiotic microbiota.

The *Cutibacterium acnes* of the present invention can promote the expression of barrier-related molecules in keratinocytes, thereby promoting the maintenance of skin barrier function. In certain examples of the present invention, the present invention provides a use of at least one of the *Cutibacterium acnes,* the cellular component thereof, the metabolite thereof, the derivative of the metabolite thereof, or the secretion thereof as mentioned above in the preparation of a promoter of the expression of a skin tissue barrier-related molecule; the skin tissue barrier-related molecule includes at least one of Filaggrin, loricrin, and involucrin. In certain examples of the present invention, the present invention provides a use of at least one of the *Cutibacterium acnes,* the cellular component thereof, the metabolite thereof, the derivative of the metabolite thereof, or the secretion thereof as mentioned above in the preparation of a skin-improving product for enhancing skin barrier function.

The *Cutibacterium acnes* of the present invention can inhibit skin pruritus by suppressing the activation of dorsal root ganglion cells; the inhibition of pruritus reflects in the inhibition of acute and chronic pruritus caused by histamine, IL-4, etc., as well as the suppression of ion influx and action potentials in dorsal root ganglion cells. In certain examples of the present invention, the present invention provides a use of at least one of the *Cutibacterium acnes,* the cellular component thereof, the metabolite thereof, the derivative of the metabolite thereof, or the secretion thereof as mentioned above in a neural cell inhibitor. In certain examples of the present invention, the present invention provides a use of at least one of the *Cutibacterium acnes,* the cellular component thereof, the metabolite thereof, the derivative of the metabolite thereof, or the secretion thereof as mentioned above in the preparation of a skin-improving product for inhibiting skin pruritus.

The *Cutibacterium acnes* of the present invention is also capable of repairing skin damage. In certain examples of the present invention, the present invention provides a use of at least one of the *Cutibacterium acnes,* the cellular component thereof, the metabolite thereof, the derivative of the metabolite thereof, or the secretion thereof as mentioned above in a skin-improving product for repairing skin damage.

The *Cutibacterium acnes* as mentioned above has a use in the preparation of a skin-improving product for at least one of: treating an inflammation-related skin disease, promoting the balance and homeostasis of skin symbiotic microbiota, enhancing skin barrier function, repairing skin damage, or suppressing skin pruritus; specifically, the skin-improving product is a medicament or a skincare product.

In the use of the *Cutibacterium acnes* as mentioned above, the application concentration, dilution ratio, duration of action, and frequency of the *Cutibacterium acnes* may vary based on different diseases and specific individual circumstances; the supernatant (metabolic products) obtained from the *Cutibacterium acnes* cultivated for different durations, its dilution ratio, topical concentration, duration of action, and frequency may also alter according to different inflammatory skin diseases and specific individual circumstances. The *Cutibacterium acnes* of the present invention, in addition to the mechanisms of action of high yield of propionic acid, inhibiting *Staphylococcus aureus* proliferation, enhancing the skin barrier, and inhibiting pruritus, may also have other potential protective mechanisms, such as producing specific antimicrobial metabolites and modulating host immunity.

The present invention provides a composition comprising at least one of the *Cutibacterium acnes,* the cellular component thereof, the metabolite thereof, the derivative of the metabolite thereof, or the secretion thereof as mentioned above , and an excipient. In certain examples of the present invention, the excipient includes at least one of protectants, humectants, emollients, abrasives, salts, and topical formulations. In one example, the excipient includes protectants, humectants, emollients, abrasives, salts, and/or a thickened topical formulation of surfactants.

The present invention further provides a skin-improving product comprising the aforementioned composition and a carrier; the skin-improving product is a medicament or a skincare product; the carrier is a pharmaceutically acceptable carrier or an excipient acceptable for skincare products.

Here, the pharmaceutically acceptable carrier may include sugars, such as lactose, glucose, and sucrose; starches, such as corn starch and potato starch; cellulose and derivatives thereof, such as sodium carboxymethyl cellulose, ethyl cellulose, and methyl cellulose; malt; gelatin; plant oil, peanut oil, corn oil, cocoa butter, and sesame oil; polyols, such as glycerol, sorbitol, and mannitol; flavoring agents; tablet excipients; stabilizers; isotonic saline solutions, such as physiological saline; and phosphate-buffered saline. The skincare product provide functions such as moisturizing, repairing, restoring barrier function, anti-aging, whitening, antioxidant, and sun protection. Besides the aforementioned composition, the skincare product is not particularly limited in other components and may include an excipient with moisturizing, whitening, desensitizing, and soothing effects, as well as an excipient such as texture modifiers, preservatives, and thickeners, etc. Those skilled in the art may select according to the formulation of the skincare product. In certain examples of the present invention, the formulations of the medicament are creams, ointments, unguents, salves, sprays, powders, medicinal oils, thickened formulations, or poultices. In certain examples of the present invention, the formulations of the skincare product are aqueous solutions, oil-based solutions, or gels.

The present invention further provides a method for isolating and culturing the *Cutibacterium acnes* as mentioned above, comprising the following steps:
Step 1: collecting skin microbiota from the perilesional areas of atopic dermatitis patients.
Step 2: selecting *Cutibacterium acnes* from the skin microbiota collected in Step 1 and culturing under anaerobic conditions to obtain purified *Cutibacterium acnes.*
Step 3: adjusting the turbidity of the purified *Cutibacterium acnes* obtained in Step 2 to 0.5 to 1.0 McFarland units in broth containing 2% to 10% glycerol, and then culturing for growth until the mid-logarithmic growth phase to obtain the *Cutibacterium acnes* of the present invention.

The present invention initially involves collecting skin microbiota from the perilesional areas of atopic dermatitis patients. Specifically, skin microbiota is collected from the cheek, axilla, or forearm perilesional areas of atopic dermatitis patients, and is immediately inoculated onto a culture medium, streaked for isolation, and cultured under anaerobic conditions at 37°C for 3 to 4 days.

After collecting a skin microbiota, the present invention involves selecting *Cutibacterium acnes* from the skin microbiota, followed by culturing under anaerobic conditions to obtain purified *Cutibacterium acnes.* Specifically, single colonies morphologically resembling *Cutibacterium acnes* are randomly selected from the skin microbiota and inoculated onto another culture medium, placed in an anaerobic environment and cultured at 37°C for 3 to 4 days. The single colonies of *Cutibacterium acnes* are then selected by detection, inoculated onto another culture medium, placed in an anaerobic environment at 37°C for another 3 to 4 days until the third generation to obtain purified *Cutibacterium acnes.* In one example, the method for the detection includes matrix-assisted laser desorption/ionization time-of-flight mass spectrometry, 16S rDNA sequencing, or whole-genome sequencing.

After obtaining the purified *Cutibacterium acnes,* the present invention involves adjusting the turbidity to 0.5 to 1.0 McFarland units in broth containing 2 to 10% glycerol, followed by cultivation for growth to the mid-logarithmic growth phase to obtain the *Cutibacterium acnes* of the present invention. Specifically, the present invention involves taking a small amount of purified *Cutibacterium acnes* to be adjusted to a turbidity of 0.5 to 1.0 McFarland units in broth containing 2 to 10% glycerol. The culture is placed in an anaerobic environment and cultured at 37°C for 20 to 25 hours, allowing growth to the mid-logarithmic phase to obtain the *Cutibacterium acnes* of the present invention. The *Cutibacterium acnes* is subjected to centrifugation and the supernatant is collected, and cryopreserved at -80°C to -100°C. In one example, the broth is at least one of Brucella broth or brain heart infusion broth.

The present invention provides the *Cutibacterium acnes,* a use, a composition, and a medicament thereof. By isolating and purifying skin microbiota from clinical samples of skin microbiota, the present invention identifies a *Cutibacterium acnes* AD3 strain with high yield of propionic acid by detecting the culture supernatant of *Cutibacterium acnes* using targeted SCFA-targeting metabolic mass spectrometry. The strain is derived from the skin microbiota of atopic dermatitis patients and demonstrates good safety and colonization performance for long-term topical use. When applied to the skin surface, it sustainably secretes metabolic products such as propionic acid, effectively treating inflammatory skin diseases, maintaining the homeostasis of the skin symbiotic microbiota, and promoting long-term skin health. Skin-improving products prepared with this strain are used to treat skin lesions in patients with inflammatory skin-related diseases, which is safe and effective, and is capable of promoting skin barrier function, suppressing pruritus, maintaining the homeostasis of skin symbiotic microbiota, and promoting long-term skin health. Experimental results indicate that the *Cutibacterium acnes* of the present invention has high yield of propionic acid. After 24 hours of anaerobic cultivation, the supernatant containing 100,000 CFU of the strain of the present invention contained 291.695 µg/mL of propionic acid, which can significantly inhibit inflammatory factors, achieving the effect of treating inflammatory skin-related diseases; it can also promote the expression of barrier-related molecules, inhibit the proliferation of *Staphylococcus aureus,* suppress pruritus, and repair skin damage.

### BIOLOGICAL DEPOSIT STATEMENT

Biological material: *Cutibacterium acnes* AD3, taxonomic designation: *Cutibacterium acnes.* was deposited on July 15, 2022, with the Guangdong Microbial Culture Collection Center (GDMCC), located at 5th Floor, Building 59, Courtyard 100, Xianliezhong Road, Guangzhou, Institute of Microbiology, Guangdong Academy of Sciences, and the deposit accession number is GDMCC No: 62625.

### BRIEF DESCRIPTION OF THE DRAWINGS

FIG. 1: Graph of the content of propionic acid in the supernatant of *Cutibacterium acnes* strain.
FIG. 2: Diagram of the effect of topical *Cutibacterium acnes* AD3 strain on AD-like dermatitis mice.
FIG. 3: Graph of scratching frequency data in mice.
FIG. 4: Graph of comparison of serum IgE levels in mice.
FIG. 5: Expression map of various inflammatory cytokines in murine auricular tissue cells.
FIG. 6: Expression map of skin barrier-related molecules in mice.
FIG. 7: Graph of comparison of transepidermal water loss and stratum corneum hydration in murine auricular skin.
FIG. 8: Diagram of the effect of topically applied *Cutibacterium acnes* AD3 strain on psoriasis-like dermatitis mice.
FIG. 9: Diagram of the effect of topically applied *Cutibacterium acnes* AD3 strain on contact dermatitis-like mice.
FIG. 10: Diagram of the effect of the topically applied metabolic products of *Cutibacterium acnes* AD3 strain on AD-like dermatitis mice.
FIG. 11: Graph of scratching frequency data in AD-like dermatitis mice.
FIG. 12: Graph of serum IgE level data in AD-like dermatitis mice.
FIG. 13: Expression map of various cytokines in the auricular tissue of AD-like dermatitis mice.
FIG. 14: Gene expression map of IL-33 in skin lesions of mice in different groups.
FIG. 15: Western-blot analysis diagram of human primary keratinocytes stimulated with different concentrations of propionic acid.
FIG. 16: Differential gene expression, mRNA expression, and protein expression maps of IL-33 in keratinocytes after silencing HDAC2 by siRNA.
FIG. 17: Differential gene expression, mRNA expression, and protein expression maps of IL-33 in keratinocytes after silencing HDAC3 by siRNA.
FIG. 18: Differential gene expression map of AhR, AhRR, and CYP1A1 in keratinocytes after propionic acid stimulation.
FIG. 19: Differential gene expression map of AhR in keratinocytes after silencing HDAC2 and HDAC3.
FIG. 20: Acetylation level diagram of the AhR promoter region in keratinocytes after propionic acid stimulation.
FIG. 21: Schematic diagram of AhR intracellular localization in keratinocytes after 6h, 12h, and 24h of propionic acid stimulation.
FIG. 22: Gross phenotype, HE staining of ear skin lesions in mice, and IL-33 immunohistochemistry staining diagram of ear skin lesions in AhR knockout mice (AhR KO) topically applied with propionic acid.
FIG. 23: Diagram of the effect of propionic acid on acute pruritus in mice induced by histamine, compound 48/80, and chloroquine.
FIG. 24: AEW modeling and propionic acid treatment regimen, along with statistical diagram of scratch times in each group of mice during the modeling period.
FIG. 25: Appearance and HE staining photographs of dorsal skin lesions in mice.
FIG. 26: Statistical diagram of epidermal thickness in each group of mice.
FIG. 27: Distribution map of epidermal nerve fibers labeled with PGP9.5 and DAPI by immunofluorescence staining.
FIG. 28: Calcium influx curves of DRG responses to IL-4, histamine, and chloroquine before and after propionic acid treatment.
FIG. 29: Gross photo of the dorsum of acne mice.
FIG. 30: HE staining of dorsal skin lesion sections in acne mice.
FIG. 31: Relative expression levels of various cytokines in the back skin lesions of acne mice by RT-qPCR.
FIG. 32: Gross photograph of the dorsum of alopecia areata mice.
FIG. 33: HE staining of dorsal skin lesion sections in alopecia areata mice.
FIG. 34: Difference in hair growth scores at different time points in alopecia areata mice from Step 1.
FIG. 35: Difference in hair growth scores at different time points in alopecia areata mice from Step 2.
FIG. 36: Ratio of follicles number in anagen phase/telogen phase in each group from Step 1.
FIG. 37: Ratio of follicles number anagen phase/telogen phase in each group from Step 2.
FIG. 38: Gross photograph of the dorsum of androgenic alopecia mice.

### DETAILED DESCRIPTION OF THE PREFERRED EMBODIMENT

The present invention discloses a *Cutibacterium acnes,* a use thereof, a composition thereof, and a medicament thereof. One skilled in the art may refer to the content disclosed herein to appropriately modify process parameters for implementation. It is particularly noted that all such substitutions and modifications are apparent to those skilled in the art and are considered to be encompassed within the ambit of the present invention. The methods and applications of the present invention have been described through preferred examples, and it is clear that those skilled in the art can make modifications, appropriate changes, or combinations of the methods and applications disclosed herein without departing from the content, spirit, and scope of the present invention in order to implement and apply the technology of the present invention.

The following further explains the present invention with reference to the examples:

### Example 1

Strain acquisition and cultivation were firstly performed: In this embodiment, skin microbiota samples were collected from the cheek, elbow, and forearm areas of 12 healthy control subjects and from the cheek, elbow, and the skin lesion surrounding areas of 11 atopic dermatitis patients. Sterile cotton swabs moistened with physiological saline were used to scrape the skin microbiota. The cotton swabs were immediately inoculated onto Columbia blood agar media plates (Wenzhou Kont Biotechnology Co., Ltd.), and a sterile disposable inoculating loop was used for streak isolation. The blood agar plates were then immediately placed in an anaerobic bag (GENbag, BioMérieux) or anaerobic jar (GasPak^{™}, BD), with an anaerobic packet (BioMérieux) added. The bag was sealed and incubated at 37°C for 3 to 4 days.

On the blood agar plate, a single colony morphologically similar to *Cutibacterium acnes* was randomly selected and inoculated onto a Columbia blood agar media plate. The blood agar plate was then placed into an anaerobic bag (GENbag, BioMérieux) or an anaerobic jar (GasPak^{™}, BD), an anaerobic packet (BioMérieux) was added, sealed, and cultured at 37°C for 3 to 4 days. A single colony was then selected for identification using matrix-assisted laser desorption/ionization time-of-flight mass spectrometry (VITEK^{®} MALDI-TOF-MS, BioMérieux). After identifying the colony as *Cutibacterium acnes,* a nearby single colony was selected and inoculated onto a Columbia blood agar media plate. The plate was placed immediately into an anaerobic bag or an anaerobic jar, and an anaerobic packet was added. The bag was sealed, and cultured at 37°C for 3 to 4 days to obtain the third generation.

For strain purification then, a small amount of the *Cutibacterium acnes* strain for purification was scraped from the blood agar plate using a sterile cotton swab and inoculated into Brucella broth (BD) containing 2% glycerol. The turbidity was measured using a turbidimeter (Sensititre, Thermo) to achieve a turbidity of 0.5 McFarland standard, resulting in a bacterial suspension after turbidity. For each subject and each skin site, one *Cutibacterium acnes* strain was selected. 4 mL of the bacterial suspension after turbidity were transferred to a sterile, enzyme-free 5 mL screw-cap plastic centrifuge tube (Axygen), and immediately placed it into an anaerobic bag. An anaerobic packet was added, and cultured at 37°C for 24 hours. After 24 hours, when the growth reached the mid-log phase, the culture supernatant was collected by centrifugation at 2000 rpm for 10 minutes, and stored in a 2 mL sterile screw-cap plastic centrifuge tube (Axygen) at -80°C.

### Example 2

A total of 34 *Cutibacterium acnes* strains from the skin of healthy individuals and 32 *Cutibacterium acnes* strains from the skin lesions of patients with atopic dermatitis were selected and isolated. The targeted short-chain fatty acids (SCFAs) metabolomics analysis was performed with the culture supernatants. The detection method is as follows:

Appropriate amount of pure standards of acetic acid, propionic acid, butyric acid, isobutyric acid, valeric acid, isovaleric acid, and hexanoic acid were measured and prepared with ether into ten mixed standard concentration gradients: 0.02 µg/mL, 0.1 µg/mL, 0.5 µg/mL, 1 µg/mL, 2 µg/mL, 5 µg/mL, 10 µg/mL, 25 µg/mL, 50 µg/mL, and 100 µg/mL. The stock solutions and working standard solutions were stored at -20°C. Samples were taken out and transferred to 2 mL centrifuge tubes. To each tube, 50 µL of 15% phosphoric acid, 10 µL of internal standard solution (75 µg/mL), and 140 µL of diethyl ether were added, followed by vortex mixing for 1 minute. The internal standard solution used was isocaproic acid. The samples were then centrifuged at 12,000 rpm 4°C for 10 minutes. The supernatant was collected and subjected to analysis (Gas Chromatograph: Thermo, Trace 1300; Mass Spectrometer: Thermo, ISQ 7000).

The gas chromatograph conditions were set as follows: Agilent HP-INNOWAX chromatographic column; capillary column with a size of 30 m × 0.25 mm ID × 0.25 µm; split injection, and injection volume of 1 µL, split ratio of 10:1; injector temperature at 250°C; ion source temperature at 300°C; transfer line temperature at 250°C; temperature for program heating up starting at 90°C, then ramped at 10°C/min to 120°C, followed by a ramp at 5°C/min to 150°C, and finally a ramp at 25°C/min to 250°C, maintained for 2 minutes; carrier gas: helium, flow rate 1.0 mL/min. The mass spectrometer conditions were set as follows: electron ionization (EI) source, SIM scan mode, electron energy at 70 eV.

Each working standard solution was analyzed by LC-MS, with the concentration of the working standard solution as the x-axis and the ratio to the internal standard peak area as the y-axis. The linear range was investigated, and a standard curve was plotted. Based on the established sample pretreatment and instrument analysis methods, quantitative analysis was performed on all samples, and the contents (µg/mL) of acetic acid, propionic acid, butyric acid, isobutyric acid, valeric acid, isovaleric acid, and hexanoic acid were determined.

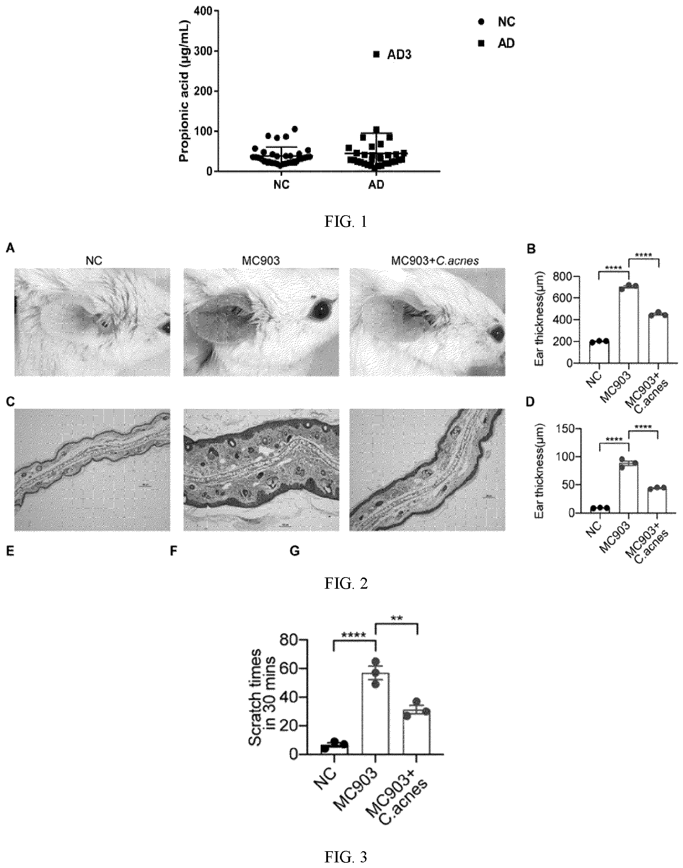

The propionic acid content in the culture supernatants of all 66 *Cutibacterium acnes* strains was analyzed, with the results shown in FIG. 1 and Table 1. FIG. 1 is the plot of propionic acid content in the supernatant of *Cutibacterium acnes* strain, where NC refers to normal healthy individuals and AD refers to patients with atopic dermatitis. Table 1 presents the propionic acid content in the culture supernatants of all 66 *Cutibacterium acnes* strains, where NC refers to strains isolated from healthy individuals, AD refers to strains isolated from patients with atopic dermatitis, FA refers to strains isolated from the cheek, AM refers to strains isolated from the forearm, and AF refers to strains isolated from the elbow.

**Table 1**

| Strain name (isolated from healthy individuals) | Propionic acid content in supernatant (µg/mL) | Strain name (isolated from atopic dermatitis patients) | Propionic acid content in supernatant (µg/mL) |
|---|---|---|---|
| NC1-FA | 35.609 | AD1-FA | 24.697 |
| NC1-AF | 23.446 | AD1-AF | 28.798 |
| NC1-AM | 25.840 | AD1-AM | 14.224 |
| NC2-FA | 23.737 | AD2-FA | 104.026 |
| NC2-AF | 21.150 | AD2-AF | 61.324 |
| NC2-AM | 20.596 | AD2-AM | 23.386 |
| NC3-FA | 23.933 | AD3-FA | 291.695 |
| NC3-AF | 24.995 | AD3-AF | 58.820 |
| NC3-AM | 31.968 | AD3-AM | 41.075 |
| NC4-FA | 37.375 | AD4-FA | 34.840 |
| NC5-FA | 36.661 | AD4-AF | 13.265 |
| NC5-AF | 38.582 | AD4-AM | 16.219 |
| NC5-AM | 47.747 | AD5-FA | 84.775 |
| NC6-FA | 52.889 | AD5-AF | 45.487 |
| NC6-AF | 35.710 | AD5-AM | 39.906 |
| NC6-AM | 33.785 | AD6-FA | 84.615 |
| NC7-FA | 38.238 | AD6-AF | 28.341 |
| NC7-AF | 42.408 | AD6-AM | 68.292 |
| NC7-AM | 105.414 | AD7-FA | 8.587 |
| NC8-FA | 21.176 | AD7-AF | 14.343 |
| NC8-AF | 18.723 | AD7-AM | 24.603 |
| NC8-AM | 21.887 | AD8-FA | 29.916 |
| NC9-FA | 31.637 | AD8-AF | 19.867 |
| NC9-AF | 88.343 | AD8-AM | 42.248 |
| NC9-AM | 86.577 | AD9-FA | 31.730 |
| NC10-FA | 57.172 | AD9-AF | 19.454 |
| NC10-AF | 35.759 | AD9-AM | 22.633 |
| NC10-AM | 83.554 | AD10-FA | 40.077 |
| NC11-FA | 31.303 | AD10-AF | 28.148 |
| NC11-AF | 33.861 | AD10-AM | 19.389 |
| NC11-AM | 43.824 | AD11-FA | 45.424 |
| NC12-FA | 14.304 | AD11-AF | 29.377 |
| NC12-AF | 17.838 | / | / |
| NC12-AM | 17.509 | / | / |

As shown in FIG. 1 and Table 1, *Cutibacterium acnes* strain, numbered as AD3 strain-FA, isolated from the facial skin microbiota swabs of atopic dermatitis patients, exhibited the highest propionic acid content in the supernatant, measuring 291.695 µg/mL. This demonstrates that the present invention successfully screened for *Cutibacterium acnes* AD3 strain, with a high yield of propionic acid.

### Example 3

It is proved by experiments that the inhibitory effect of topically applied *Cutibacterium acnes* AD3 strain on atopic dermatitis (AD)-like dermatitis. The treatment regimen with *Cutibacterium acnes* AD3 strain applied topically was as follows:

2 nmol of calcipotriol (MC903) was applied to the auricular of a mouse to induce an AD-like dermatitis mouse model. Meanwhile, *Cutibacterium acnes* AD3 strain was applied topically at a daily dose of 1×10⁵ CFU/mL for 14 days.

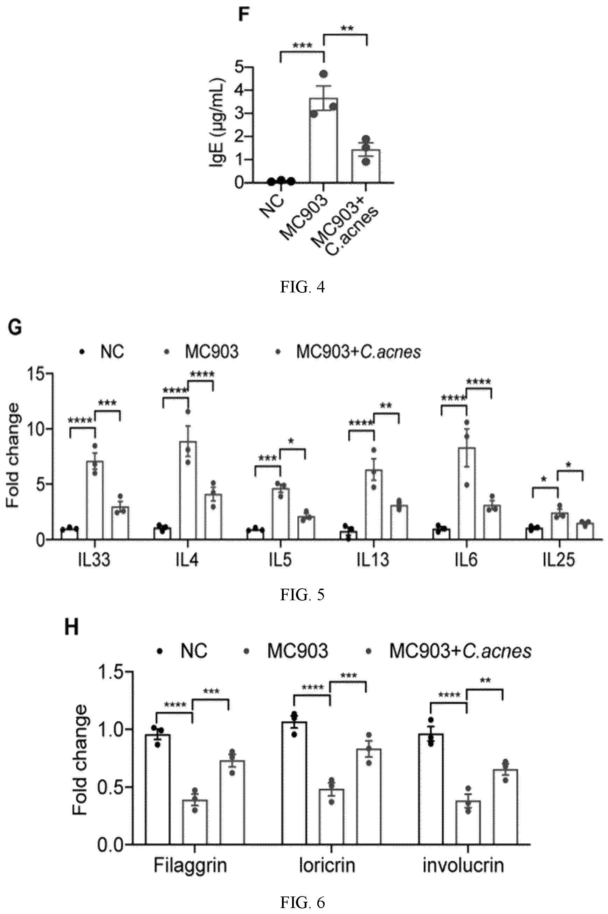

Following the application of AD3 strain, the AD-like inflammation in the mice was significantly alleviated, and the results are shown in FIG. 2. FIG. 2 is the effect diagram of topical *Cutibacterium acnes* AD3 strain on AD-like dermatitis mice. Specifically, A of FIG. 2 is the gross photograph of the mice; B of FIG.2 is a schematic of gross ear thickness of the mice; C of FIG. 2 is the HE staining diagram of the auricle sections of the mice; D of FIG. 2 is a schematic of epidermal thickness in the auricular of the mice. From A-D of FIG. 2, it is clear that the topical application of *Cutibacterium acnes* AD3 strain significantly improved the AD-like dermatitis in the mice. The scratching frequency of the mice was statistically evaluated, and the results are shown in FIG. 3, and FIG. 3 is the data graph presenting the scratching frequency. The serum IgE levels of the mice were measured, and the results are shown in FIG. 4, and FIG.4 is a comparison graph for serum IgE levels. Additionally, cytokine expression in the auricular tissue of the mice was assessed, and the results are presented in FIG. 5, and FIG.5 is the expression map for each inflammatory cytokines in the auricular tissue cells. FIGs. 3-5 further illustrate the effect of topical application of *Cutibacterium acnes* AD3 strain in alleviating AD-like dermatitis symptoms. Furthermore, the data regarding the gross ear thickness, epidermal thickness of the auricle, scratching frequency, serum IgE levels, and the expression of inflammatory cytokines in the auricular tissue of the mice are summarized in Table 2.

**Table 2**

| Indicators | Parameters | NC | MC903 | MC903+*C*. *acnes* |
|---|---|---|---|---|
| Gross ear thickness (µm) | MEAN | 199.67 | 704.83 | 449.83**** |
| | SEM | 3.57 | 9.67 | 7.87 |
| Epidermal thickness of ear (µm) | MEAN | 8.71 | 88.40 | 43.97**** |
| | SEM | 0.21 | 3.49 | 0.76 |
| Scratching frequency | MEAN | 6.67 | 57.00 | 31.33** |
| | SEM | 1.19 | 3.77 | 2.42 |
| Mice serum IgE (µg/ml) | MEAN | 0.08 | 3.66 | 1.44** |
| | SEM | 0.01 | 0.43 | 0.23 |
| IL33 | MEAN | 0.99 | 7.09 | 2.96*** |
| | SEM | 0.05 | 0.60 | 0.39 |
| IL4 | MEAN | 1.06 | 8.88 | 4.10**** |
| | SEM | 0.13 | 1.13 | 0.51 |
| IL5 | MEAN | 0.92 | 4.61 | 2.09* |
| | SEM | 0.06 | 0.30 | 0.20 |
| IL13 | MEAN | 0.75 | 6.31 | 3.10** |
| | SEM | 0.28 | 0.80 | 0.19 |
| IL6 | MEAN | 0.99 | 8.28 | 3.11**** |
| | SEM | 0.13 | 1.40 | 0.33 |
| IL25 | MEAN | 1.04 | 2.40 | 1.48* |
| | SEM | 0.09 | 0.28 | 0.10 |

In Table 2, the statistical differences between the MC903 group and the MC903 + C. *acnes* group are as follows: *, P-value < 0.05; **, P-value < 0.01; ***,P-value < 0.001; ****,P -value< 0.0001.

As shown in FIGs. 2-5 and Table 2, topical application of *Cutibacterium acnes* AD3 strain significantly alleviated AD-like inflammation in the mice. General observations showed that the MC903 + *C. acnes* group exhibited reduced erythema, fewer scales, and a significant decrease in ear thickness. HE staining also indicated a reduction in epidermal thickness and a decrease in the infiltration of inflammatory cells in the dermis. Additionally, the topical application of AD3 strain could significantly inhibit scratching behavior in the mice, suppress the expression of inflammatory cytokines IL-33, IL-4, IL-5, IL-6, IL-13, and IL-25, and reduced the total IgE levels in peripheral blood.

The topical application of AD3 strain contributed to the maintenance of skin barrier function, as shown in FIG. 6 and Table 3. FIG. 6 is the expression map of skin barrier-related molecules in the mice.

**Table 3**

| Indicators | Parameters | NC | MC903 | MC903+*C*. *acnes* |
|---|---|---|---|---|
| Filaggrin | MEAN | 0.96 | 0.39 | 0.96*** |
| | SEM | 0.04 | 0.04 | 0.05 |
| loricrin | MEAN | 1.06 | 0.48 | 0.96*** |
| | SEM | 0.04 | 0.05 | 0.06 |
| involucrin | MEAN | 0.96 | 0.38 | 0.96** |
| | SEM | 0.05 | 0.05 | 0.04 |

In Table 3, the statistical differences between the MC903 group and the MC903 + C. *acnes* group are as follows: *, P-value< 0.05;**, P-value < 0.01; ***, P-value < 0.001.

As shown in FIG. 6 and Table 3, the expression of skin barrier-related molecules in AD-like dermatitis mice was substantially reduced compared with healthy mice. However, after the topical application of AD3 strain, the expression of skin barrier-related molecules was promoted to increase.

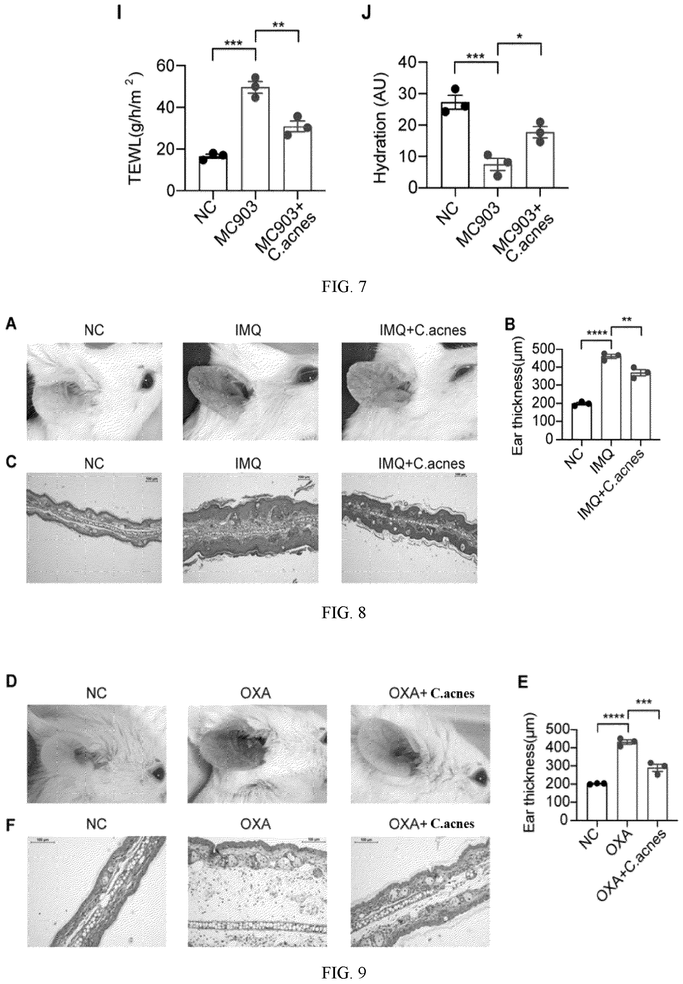

The transepidermal water loss (TEWL) condition and stratum corneum hydration of the auricular skin of the mice were also tested, and the results are shown in FIG. 7 and Table 4. FIG. 7 is a comparison graph of the TEWL and stratum corneum hydration of the murine auricular skin. The results in FIG. 7 and Table 4 further confirm that the topical application of AD3 strain can promote the maintenance of skin barrier function.

**Table 4**

| Indicators | Parameters | NC | MC903 | MC903+*C*. *acnes* |
|---|---|---|---|---|
| Transepidermal water loss (g/h/m²) | MEAN | 16.58 | 49.67 | 30.85** |
| | SEM | 0.79 | 2.28 | 2.14 |
| Stratum corneum hydration (AU) | MEAN | 27.20 | 7.45 | 17.69* |
| | SEM | 1.80 | 1.60 | 1.50 |

In Table 4, the statistical differences between the MC903 group and MC903 + C. *acnes* group are: *, P-value < 0.05; **, P-value < 0.01.

Additionally, in FIGs. 2 to 7, NC refers to normal healthy mice; MC903 refers to AD-like dermatitis mice; MC903 + *C. acnes* refers to AD-like dermatitis mice treated with topical application of AD3 strain.

In summary, the topical application of C. *acnes* AD3 strain promoted the expression of skin barrier-related molecules such as Filaggrin, Loricrin, and Involucrin, promoted skin stratum corneum hydration, and reduced the level of trans-epidermal water loss (TEWL), suggesting that topical application of AD3 strain plays a role in the regulation of skin barrier function.

### Example 4

It is demonstrated by experiments that the inhibitory effect of topical application of AD3 strain on other inflammatory skin diseases. The topical application regimen for *C. acnes* AD3 strain is as follows:
*Cutibacterium acnes* AD3 strain was topically applied at a daily dose of 1 × 10⁵ CFU/mL to the skin of psoriasis-like dermatitis mice and contact dermatitis-like mice, respectively. Specifically, psoriasis-like dermatitis mice were induced with IMQ (Imiquimod); contact dermatitis, mice were induced with QXA (Oxazolone).

The experimental results are shown in FIG. 8, FIG. 9, and Table 5. FIG. 8 is the effect diagram of topical application of *Cutibacterium acnes* AD3 strain on psoriasis-like dermatitis mice, and FIG. 9 is the effect diagram of topical application of *Cutibacterium acnes* AD3 strain on contact dermatitis-like mice. Specifically, A of FIG. 8 is the gross photograph of the psoriasis-like dermatitis mice, B of FIG. 8 is the gross ear thickness data diagram of psoriasis-like dermatitis mice, and C of FIG. 8 shows the HE-staining diagram of the auricular section of the psoriasis-like dermatitis mice. D of FIG. 9 is the gross photograph of the contact dermatitis-like mice, E of FIG. 9 is the gross ear thickness data diagram of contact dermatitis-like mice, and F of FIG. 9 is the HE-staining diagram of the auricular section of the contact dermatitis-like mice. In FIG.8, NC refers to normal healthy mice, IMQ refers to mice induced with IMQ, and IMQ + *C. acnes* refers to IMQ-induced mice topically treated with *Cutibacterium acnes* of the present invention. In FIG. 9, NC refers to normal healthy mice, OXA refers to mice induced with OXA, and OXA + *C. acnes* refers to OXA-induced mice topically treated with *Cutibacterium acnes* of the present invention.

**Table 5**

| Indicators | Parameters | NC | IMQ | IMQ+*C*. *acnes* |
|---|---|---|---|---|
| Gross ear thickness (µm) | MEAN | 198.67 | 459.67 | 370.00 ** |
| | SEM | 3.13 | 5.48 | 7.97 |

| Indicators | Parameters | NC | OXA | OXA+*C*. *acnes* |
|---|---|---|---|---|
| Gross ear thickness (µm) | MEAN | 202.67 | 431.33 | 289.17*** |
| | SEM | 0.87 | 5.70 | 9.21 |

In Table 5, the statistical differences are as follows: *, P-value < 0.05; **, P-value < 0.01; ***, P-value < 0.001.

As shown in FIG. 8, FIG. 9, and Table 5, topical application of AD3 strain clearly alleviated the inflammation in the mice. The general observations showed that the topical AD3 strain group exhibited reduced erythema, reduced scales, and a significant decrease in ear thickness. There was also a decrease in the inflammatory cells infiltrated in the dermis.

### Example 5

It is proved by experiments that the inhibitory effect of the metabolites of topically applied *Cutibacterium acnes* AD3 strain on AD-like dermatitis. The acquisition and topical application regimen of *Cutibacterium acnes* AD3 strain metabolites are described as follows:

1 × 10⁵ CFU/mL of AD3 strain was cultured for 24 hours at 37°C under anaerobic conditions in complete culture medium. The culture supernatant was collected and filtered through a 0.22 µm filter to obtain sterile supernatant, which represents the metabolites of AD3 strain. The sterile supernatant was then diluted five-fold and applied topically to the auricle of the inflammatory mice at a daily dose of 30 µL per ear.

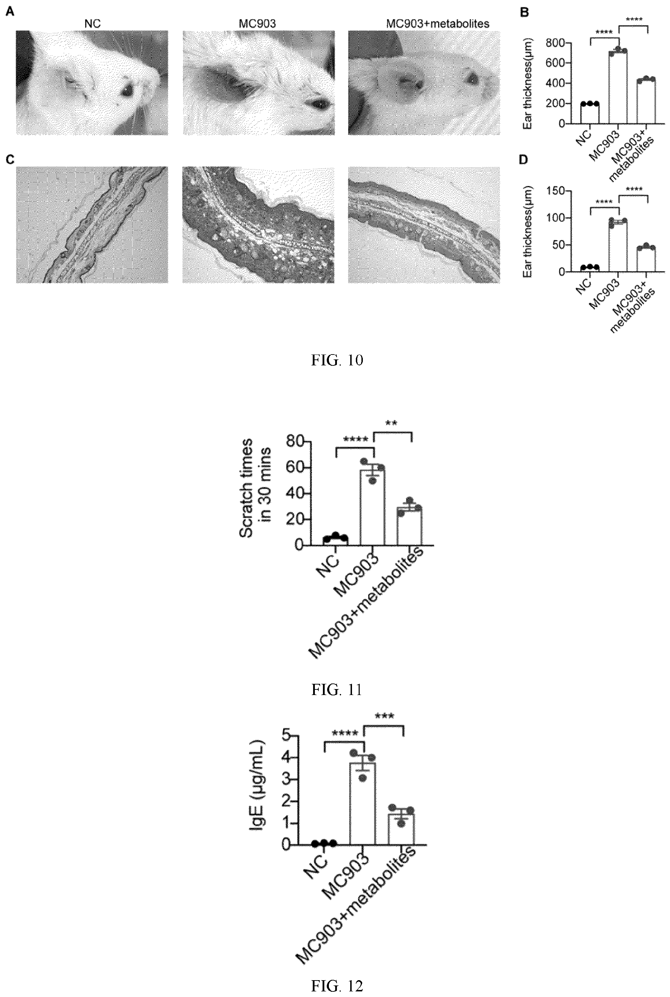

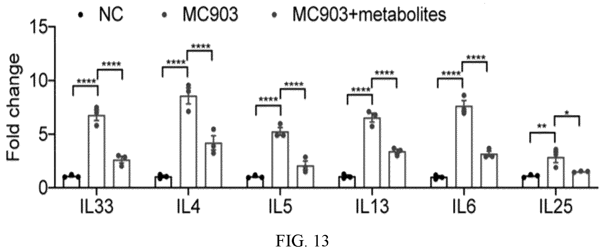

The experimental results are shown in FIGs. 10-13 and Table 6. FIG. 10 is the effect diagram of the metabolites of *C. acnes* AD3 strain applied topically to AD-like dermatitis mice. Specifically, A of FIG. 10 is the gross photograph of the AD-like dermatitis mice; B of FIG. 10 is the gross ear thickness data diagram of AD-like dermatitis mice; C of FIG. 10 is the HE-staining diagram of the auricular section of the AD-like dermatitis mice; D of FIG. 10 is the data diagram of epidermal ear thickness of the auricle of AD-like dermatitis mice; FIG. 11 is the data diagram of scratching frequency of the AD-like dermatitis mice; FIG. 12 is data diagram of the serum IgE levels of the AD-like dermatitis mice; FIG. 13 is the expression map of various cytokines in the auricular tissue of the AD-like dermatitis mice.

**Table 6**

| Indicators | Parameters | NC | MC903 | MC903+metabolites |
|---|---|---|---|---|
| Gross ear thickness (µm) | MEAN | 197.67 | 719.33 | 437.00 **** |
| | SEM | 0.68 | 7.53 | 4.19 |
| Epidermal thickness of ear (µm) | MEAN | 9.04 | 92.16 | 46.00 **** |
| | SEM | 0.20 | 1.65 | 0.84 |
| Scratching frequency | MEAN | 6.33 | 58.33 | 29.67 ** |
| | SEM | 0.42 | 2.08 | 1.37 |
| Mice serum IgE (µg/ml) | MEAN | 0.08 | 3.76 | 1.43 *** |
| | SEM | 0.01 | 0.16 | 0.11 |
| IL33 | MEAN | 1.06 | 6.76 | 2.63 **** |
| | SEM | 0.03 | 0.23 | 0.14 |
| IL4 | MEAN | 1.03 | 8.55 | 4.19 **** |
| | SEM | 0.05 | 0.35 | 0.31 |
| IL5 | MEAN | 1.02 | 5.25 | 2.09 **** |
| | SEM | 0.04 | 0.17 | 0.19 |
| IL13 | MEAN | 1.04 | 6.53 | 3.40 **** |
| | SEM | 0.05 | 0.19 | 0.10 |
| IL6 | MEAN | 0.98 | 7.61 | 3.17 **** |
| | SEM | 0.05 | 0.24 | 0.12 |
| IL25 | MEAN | 1.08 | 2.86 | 1.49 * |
| | SEM | 0.04 | 0.24 | 0.02 |

In Table 6, the statistical differences between the MC903 group and the MC903 + metabolites group are as follows: *, P-value < 0.05; **, P-value < 0.01; ***, P-value < 0.001; ****, P-value < 0.0001.

After topical application of the metabolites obtained from AD3 strain, the AD-like inflammation in mice was clearly alleviated. The general observations showed that the metabolites of topical AD3 strain group exhibited reduced erythema, fewer scales, and a clear decrease in ear thickness. There was also a decrease in the inflammatory cells infiltrated in the dermis. Additionally, the topical application of AD3 strain could clearly inhibit scratching behavior in the mice, suppress the expression of inflammatory cytokines, and reduced the total IgE levels in peripheral blood.

### Example 6

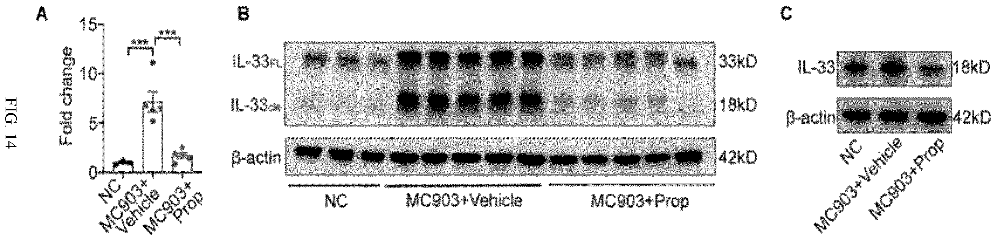

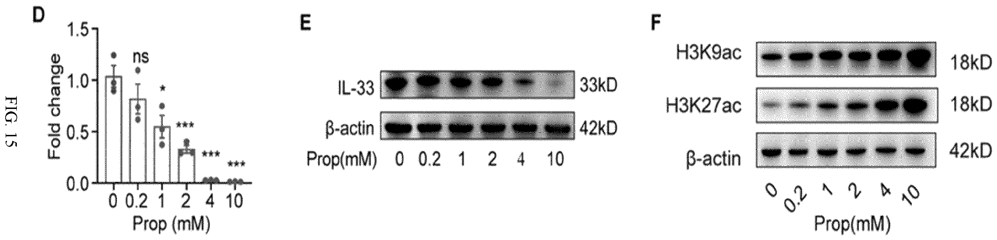

The mechanism of action of topically applied *Cutibacterium acnes* AD3 strain was investigated, confirming the mechanism behind the inhibition of AD3 strain on inflammation through its metabolic product, propionic acid:
(1) As shown in FIG. 14, and FIG. 14 is the gene expression map of IL-33 in the skin lesions of mice from each group. Specifically, A of FIG. 14 is the differential gene expression map of IL-33 in the skin lesions of mice from each group; B of FIG. 14 is the mRNA expression and protein expression map of IL-33 in the skin lesions of mice from each group; C of FIG. 14 is the expression map of IL-33 in the mouse epidermal cell line JB6 after stimulation with propionic acid. In FIG. 14, NC refers to normal healthy mice; MC903 refers to AD-like dermatitis mice; MC903 + Vehicle refers to the vehicle control group for AD-like dermatitis mice; MC903 + Prop refers to the AD-like dermatitis mice topically treated with propionic acid. As seen in FIG. 14, both *in vivo* and *in vitro* experimental results demonstrate that propionic acid inhibits the expression of the inflammatory initiator IL-33 in both murine and human primary keratinocytes.
(2) Short-chain fatty acids, such as propionic acid, are generally considered as histone deacetylase (HDAC) inhibitors in the gut. Further investigation revealed that propionic acid may also exert HDAC inhibition in the skin. The study results are shown in FIG. 15. FIG. 15 is the Western blot analysis of human primary keratinocytes stimulated with different concentrations of propionic acid; Specifically, D of FIG. 15 is the differential gene expression map of IL-33 in human primary keratinocytes stimulated with different concentrations of propionic acid. E of FIG. 15 is the mRNA expression and protein expression map of IL-33 in human primary keratinocytes after stimulation with different concentrations of propionic acid. F of FIG. 15 is a schematic representation of the acetylation level in keratinocytes after propionic acid stimulation. The Western blot results in FIG. 15 indicate that propionic acid promotes the acetylation level of histones in keratinocytes.

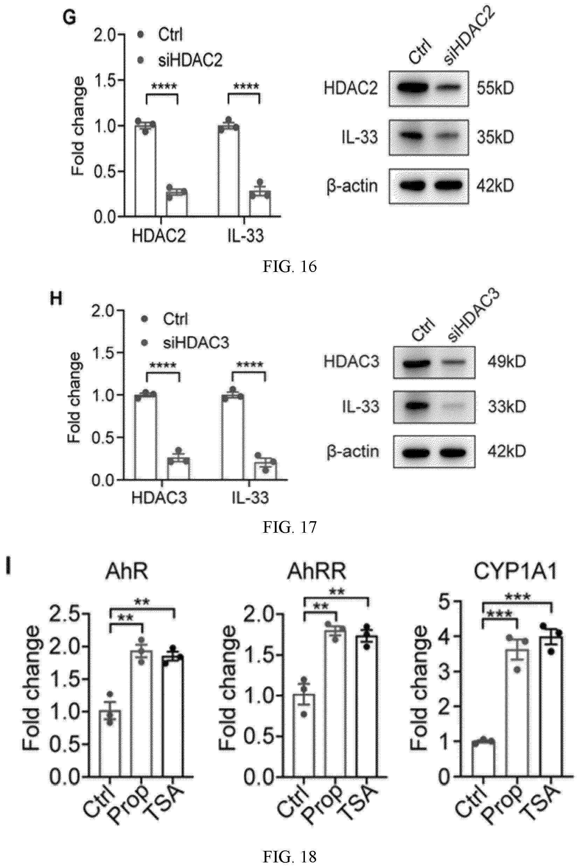

Each HDAC in keratinocytes was sequentially silenced to validate the transmission mechanism between HDACs and IL-33. The results are shown in FIGs. 16-17, where FIG. 16 is the differential gene expression, as well as mRNA expression and protein expression map of IL-33, in keratinocytes following silencing of HDAC2 with siRNA; FIG. 17 is the gene expression, mRNA expression, and protein expression map of IL-33 after silencing of HDAC3 with siRNA in keratinocytes. In FIGs. 16-17, Ctrl refers to the control group; SiHDAC2 refers to silencing HDAC2 with siRNA; SiHDAC3 refers to silencing HDAC3 with siRNA.

As shown in FIGs. 16-17, by silencing each HDAC sequentially in keratinocytes, it is found that propionic acid inhibits IL-33 expression through the inhibition of HDAC2 and HDAC3 in keratinocytes. However, there was no significant increase in acetylation levels at the promoter region of IL-33, suggesting that there may be an intermediary factor to transmit the signal between HDAC and IL-33.

(3) Furthermore, by predicting the transcription factors that bind to the human IL-33 gene promoter region, it was found that the aryl hydrocarbon receptor (AhR) exhibited significant changes after stimulation with propionic acid in keratinocytes.

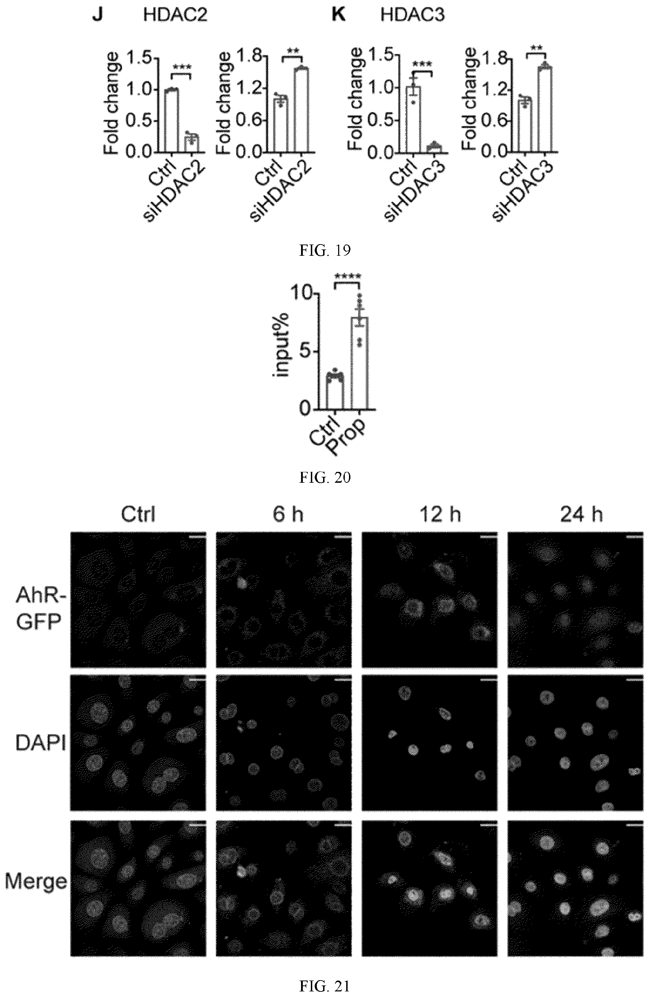

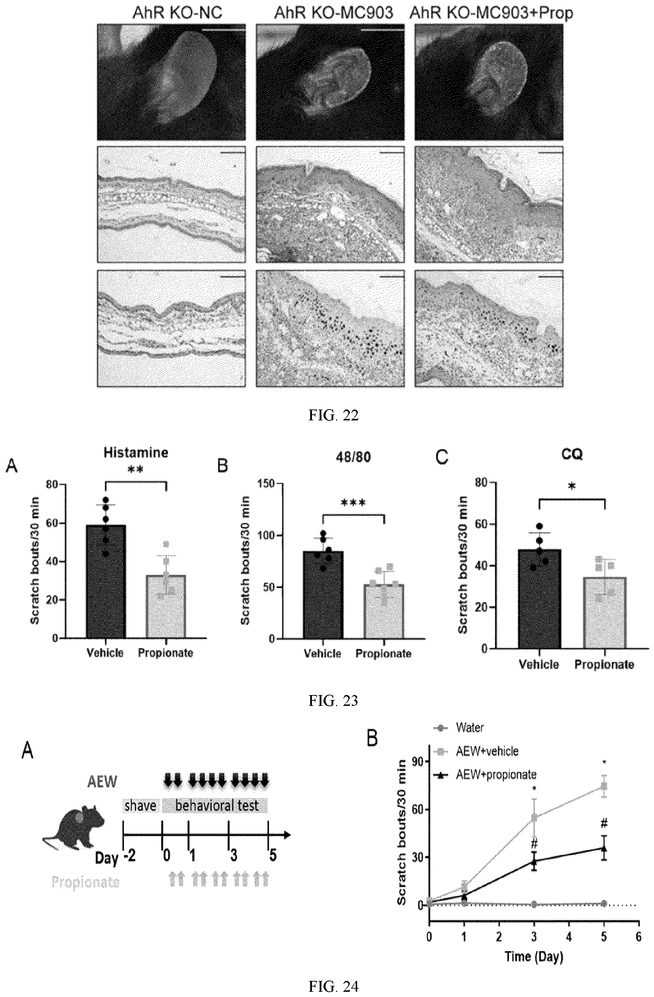

Specifically, as shown in FIGs. 18-22, FIG. 18 is the differential gene expression map of AhR, AhRR, and CYP1A1 in keratinocytes after propionic acid stimulation. FIG. 19 is the differential gene expression map of AhR in keratinocytes after silencing HDAC2 and HDAC3, with J of FIG. 19 being the differential gene expression map of AhR after silencing HDAC2, and K of FIG. 19 being the differential gene expression map of AhR after silencing HDAC3. FIG. 20 is the acetylation level of the AhR promoter region in keratinocytes following propionic acid stimulation. FIG. 21 is the intracellular localization schematic of AhR in keratinocytes at 6h, 12h, and 24h after propionic acid stimulation. FIG. 22 is the gross phenotype, HE staining of ear skin lesions in mice, and IL-33 immunohistochemistry staining of ear skin lesions in AhR knockout mice (AhR KO) topically applied with propionic acid. In FIGs. 18 to 21, Ctrl refers to the control group, Prop refers to the propionic acid group (i.e., a mice group of stimulating keratinocytes with propionic acid), and TSA refers to the trichostatin A group (i.e., a mice group of stimulating keratinocytes with trichostatin A, a known HDAC inhibitor). In FIG. 22, NC refers to normal healthy mice, MC903 refers to AD-like dermatitis mice, and MC903+Prop refers to AD-like dermatitis mice topically treated with propionic acid.

As shown in FIGs. 18 to 22, RT-qPCR proves that after stimulation with propionic acid, the expression of AhR in keratinocytes was up-regulated, and the AhR signaling pathway was activated. After silencing HDAC2 or HDAC3, the expression of AhR in keratinocytes was also found up-regulated, with an increase in acetylation at the AhR promoter region. Moreover, confocal fluorescence imaging reveals that propionic acid promotes the nuclear translocation of AhR. These findings suggest that propionic acid up-regulates AhR expression by inhibiting HDAC2/3 and may act as a ligand to bind AhR, promoting its nuclear translocation to exert its biological function. Additionally, IL-33 expression was observed up-regulated after silencing AhR in keratinocytes, and this up-regulated IL-33 expression could not be suppressed by propionic acid. In AhR knockout mice, AD-like inflammation could not be rescued by propionic acid. These results indicate that propionic acid exerts its inhibitory effect on IL-33 by up-regulating the expression and signaling of AhR.

### Example 7

The metabolic product of *Cutibacterium acnes,* propionic acid, can inhibit skin pruritus by suppressing the activation of dorsal root ganglion cells. The inhibition of pruritus is reflected in the suppression of both acute and chronic pruritus induced by histamine, IL-4, etc., as well as the inhibition of ion influx and action potentials in dorsal root ganglion cells. The specific proposal is as follows:

### (1) Effect of propionic acid on acute pruritus in mice:

The mice which had been fully adapted to the behavioral testing environment, were randomly divided into the propionic acid treatment group and the vehicle control group, with 5 to 6 wild-type C57 mice in each group. The number of scratches induced by intradermal injection of histamine, compound 48/80, and chloroquine (CQ) on the cheeks in 30 minute was recorded. In the treatment group, 10 µL of 4 mmol/L propionic acid was injected intradermally, while the control group was injected with 10 µL of PBS. 30 minutes later, different types of acute pruritogens were administered intradermally for each group, including 100 µg of histamine, 50 µg of chloroquine, and 20 µg of compound 48/80. The scratching behavior of the mice was then immediately recorded on video. The results are shown in FIG. 23, which is the effect diagram of propionic acid on acute pruritus induced by histamine, compound 48/80, and chloroquine in mice. A of FIG. 23 is the effect of histamine on acute pruritus in mice. B of FIG. 23 is the effect of compound 48/80 on acute pruritus in mice. C of FIG. 23 is the effect of chloroquine on acute pruritus in mice. The data in FIG. 23 are represented as mean ± standard error of the mean (mean ± SEM) and analyzed using an unpaired two-tailed t-test. *: p<0.05; **: p<0.01; ***: p<0.001.

As shown in FIG. 23, in the propionic acid treatment group, the acute pruritus induced by histamine (59.06±4.29 vs 33.51±4.11), chloroquine (47.80±3.57 vs 34.62±3.83), and compound 48/80 (85.17±5.05 vs 52.57±4.74) was significant low compared to the vehicle control group. The differences were statistically significant (p < 0.01).

### (2) Effect of propionic acid on chronic pruritus in mice:

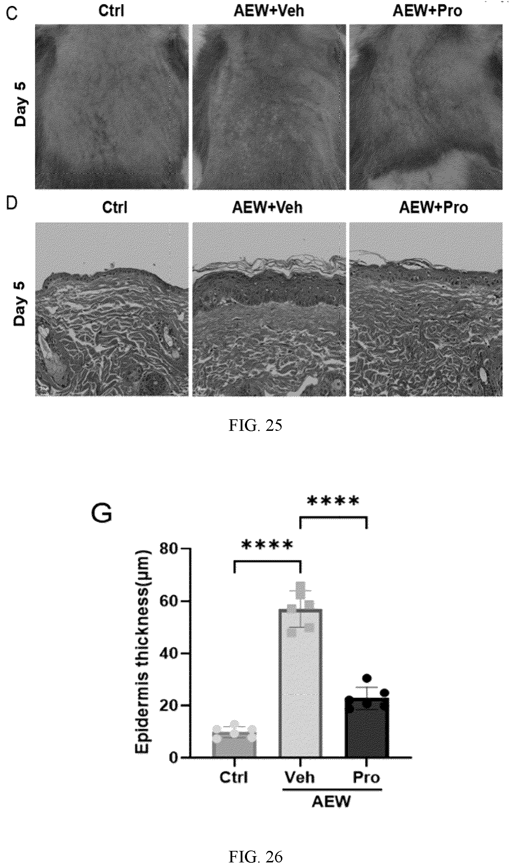

We further investigated the effect of propionic acid on pruritus in the acetone-ether-water (AEW) induced dry skin model. The results are shown in FIGs. 24 to 26. FIG. 24 is the AEW modeling and propionic acid treatment protocol, as well as the statistics of the scratching behavior of mice in each group during the modeling period. FIG. 25 is the appearance and HE staining images of the skin lesions on the backs of the mice. FIG. 26 is the statistical data of epidermal thickness in each group.

As shown in FIGs. 24 to 26, the AEW model successfully induced scratching behavior in mice, and propionic acid treatment significantly reduced the number of scratches. After modeling, skin lesions were observed with scaling and scratch marks. HE staining revealed epidermal thickening and hyperkeratosis, and propionic acid treatment significantly improved the epidermal thickness (56.95±2.84 µm vs 22.78±1.77 µm).

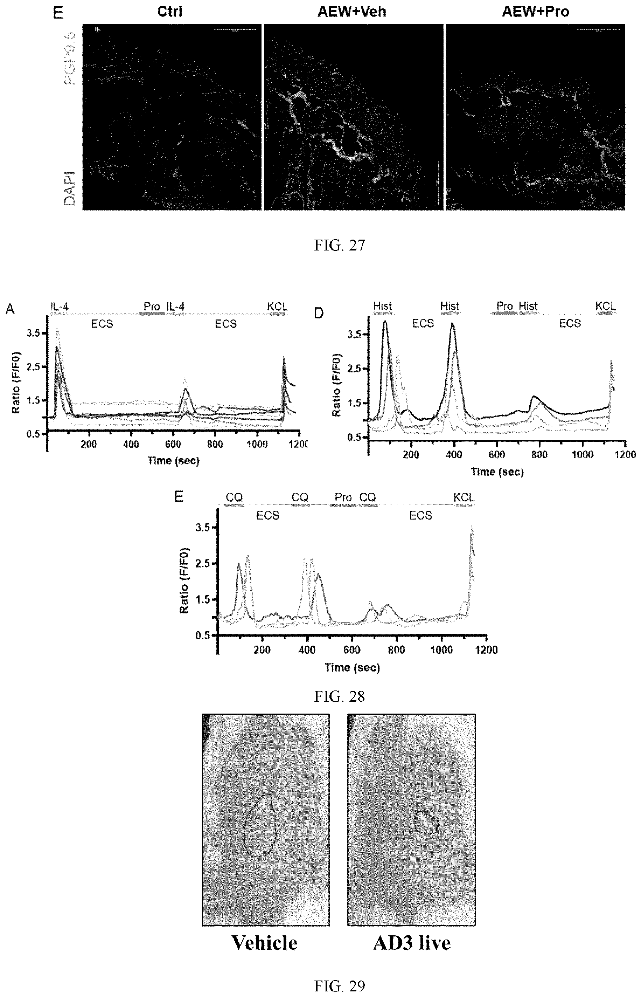

Immunofluorescence staining was used to label epidermal nerve fibers, and observation under a confocal microscope showed that in the AEW group, the number of nerve fibers sprouting from the dermis to the epidermis was increased compared to the control group. However, in the propionic acid treatment group, the distribution density of epidermal nerve fibers was reduced compared to the vehicle group, as shown in FIG. 27. FIG. 27 is the distribution map of epidermal nerve fibers labeled with PGP9.5 and DAPI through immunofluorescence staining.

### (3) Propionic acid inhibits the activation of dorsal root ganglion cells

Calcium imaging experiments were conducted to record intracellular calcium transients. IL-4, capsaicin, histamine, menthol, and chloroquine, etc., were used to perfuse for 30 seconds and the changes in calcium ion concentration were recorded. When recovering to baseline after washing with ECS, 100 µmol/L of propionic acid was used to perfuse as pre-treatment. The same pruritogens were then applied again for stimulation, and the changes of DRG response ratio and calcium ion concentration peak amplitude, were observed. The results are shown in FIG. 28. FIG. 28 is the calcium influx curves of DRG responses to IL-4, histamine, and chloroquine before and after propionic acid treatment. A of FIG. 28 is the calcium influx curve of DRG responses to IL-4 before and after propionic acid treatment; D of FIG. 28 is the calcium influx curve of DRG response to histamine; E of FIG. 28 is the calcium influx curve of DRG response to chloroquine.

As shown in FIG. 28, the Th2 inflammatory factor IL-4 induces partial calcium influx in DRGs . After incubation with propionic acid, the proportion of DRG responses to IL-4 decreased significantly, and the calcium peak amplitude also significantly reduced. In the control group incubated with vehicle, there was no noticeable change in the calcium peak values induced by IL-4 twice before and after. For acute pruritogens, it was observed that both histamine and chloroquine can induce a strong calcium influx in DRGs. The peak values of calcium transient activated by two consecutive administrations were similar. However, when the same DRG was stimulated with histamine and chloroquine after pre-incubation with propionic acid, the calcium influx induced was significantly inhibited.

In summary, the AD3 strain inhibits inflammatory factors through metabolic product, propionic acid. Specifically, propionic acid exerts anti-inflammatory effects by inhibiting the inflammatory initiator IL-33 in skin keratinocytes. This process of inhibiting IL-33 with propionic acid is achieved through promoting the expression of the aryl hydrocarbon receptor (AhR) by inhibiting HDAC2/3, and binding to propionic acid as a ligand to promote nuclear translocation of AhR.

Furthermore, the AD3 strain inhibits the proliferation of *Staphylococcus aureus* and stabilizes the balance of the skin symbiotic microbiota. In co-culture of AD3 strain and *Staphylococcus aureus in vitro,* the proliferation of *Staphylococcus aureus* was suppressed. After topical application of the AD3 strain to mice, the abundance of *Staphylococcus aureus* colonized on the skin surface decreased. The AD3 strain promotes the expression of barrier-related molecules like Filaggrin in keratinocytes, contributing to the maintenance of barrier function. Additionally, propionic acid inhibits skin pruritus in mice and suppresses the activation of cultured neural cells *in vitro.*

### Example 8: Effect of topical Cutibacterium acnes AD3 strain on an acne mouse model

The bacterial liquid of *Cutibacterium acnes* in the logarithmic growth phase was taken to turbidimetry to a density of approximately 1×10⁸ CFU/ml to 1×10⁹ CFU/ml using a turbidity meter for the animal experiments.

### 1. Preparation of artificial sebum mixture:

The artificial sebum mixture was prepared according to the following proportions: 15% of squalene; 1.2% of cholesterol; 10% of liquid paraffin; 15% of palmityl palmitate;
2.4% of oleic acid cholesterol; 1.4% of oleic acid; 2.5% of stearic acid; 2.5% of lauric acid; 5% of palmitic acid; 45% of triolein. The mixture was heated in a 70°C water bath to promote dissolution. To promote dissolution, Tween 80 was added in a 1:3 ratio (artificial sebum mixture:Tween 80), and heated in a 70°C water bath.

### 2. Topical application of Cutibacterium acnes AD3 in the acne mouse model:

BALB/c mice, aged 6-8 weeks, were depilated with a razor and depilatory cream on back, and rest for 2 days. On the back, 50 µL of *Cutibacterium acnes* ATCC6919, with a total bacterial count of approximately 1×10⁷ CFU, was injected intradermally. Afterwards, 30 µL of artificial sebum was applied to the back daily for 7 days in total. For the intervention group, 50 µL of 1×10⁸ CFU AD3 live bacteria was applied to the back daily. For the positive control group, 50 µL of sterile medium was applied to the back daily.

### 3. Topical AD3 strain on skin alleviates inflammation in the acne mouse model:

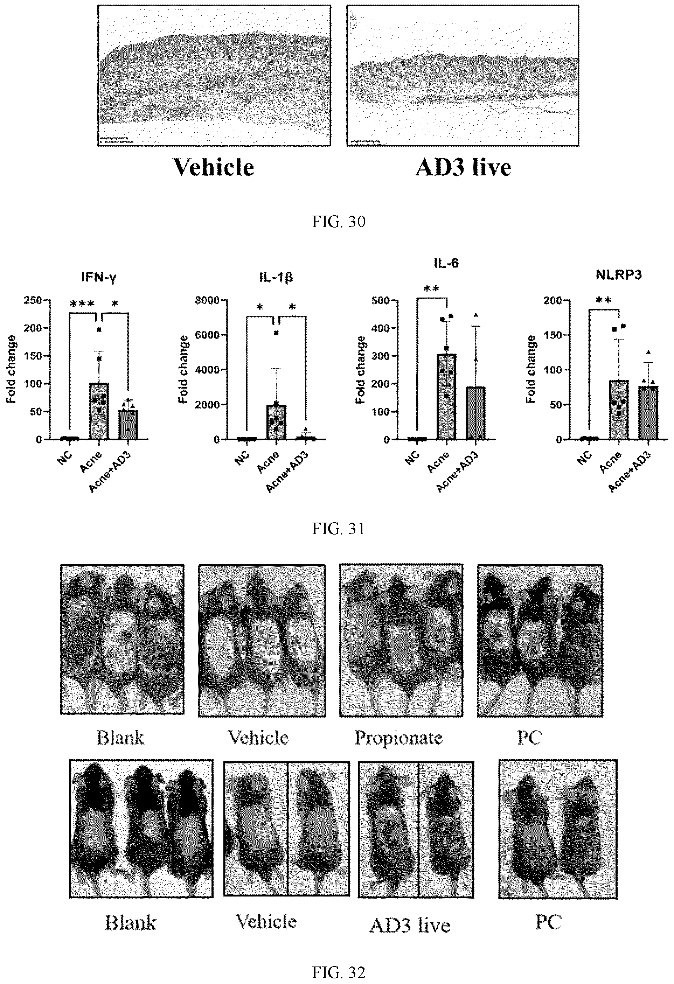

The results are shown in FIGs. 29, 30, and 31. FIG. 29 is the gross photograph of the dorsum of acne mice, wherein, Vehicle: positive control group; AD3 live: topical AD3 live bacteria group. Red circles indicate inflammatory papules. FIG. 30 is the HE staining of dorsal skin lesion sections in acne mice, wherein, Vehicle: positive control group; AD3 live: topical AD3 live bacteria group. FIG. 31 is the relative expression levels by RT-qPCR of various cytokines in the back skin lesions of acne mice, wherein, NC: blank control; Acne: positive control group; Acne+AD3: topical AD3 live bacteria group. After 7 days of topical treatment with AD3 strain, the inflammation of dorsal acne of the mice was significantly alleviated. Generally, in the topical AD3 live bacteria group, the volumes of inflammatory papules were small, the erythema and scaling on the back skin were few (FIG. 29). HE staining showed a decrease in inflammatory cells infiltrated in the dermis, and the skin thickness was thinner than that in the positive control group (FIG. 30). Moreover, the topical AD3 strain could significantly inhibit the expression of acne-related inflammatory factors such as IFN-γ and IL-1β, and could also suppress the expression of IL-6, without increasing the expression of the acne-related inflammatory factor NLRP3 (FIG. 31).

### Example 9: Effect of topical Cutibacterium acnes AD3 strain in the alopecia areata mouse model

The bacterial liquid of *Cutibacterium acnes* in the logarithmic growth phase was taken to turbidimetry to a density of approximately 1×10⁸ CFU/ml to 1×10⁹ CFU/ml using a turbidity meter for the animal experiments.

### 1. Topical application of propionic acid in the alopecia areata mouse model:

C57BL/6 mice, aged 6-8 weeks, were depilated on back using a razor and a depilatory cream, ensuring that the hair were in the telogen phase (the skin appeared pink) with the area approximately 3 cm × 4 cm. On the 9th day after hair plucking, the normal control group received an equal volume of physiological saline, while the other groups received intraperitoneal injection of cyclophosphamide (3 mg/20 g). The administration treatment started on the 2nd day after hair plucking. The model group was daily applied with 50 µl of a 1:7 mixed vehicle of propylene glycol and isopropanol. In the propionic acid treatment group, 50 µl of 1 mM sodium propionate (dissolved in the 1:7 mixed vehicle of propylene glycol and isopropyl alcohol) was applied to the depilated area. The NC group received no treatment, while 200 µl of 3% minoxidil solution was applied daily for the positive control group for 18 consecutive days. Photographs were taken regularly to observe the hair loss and growth in the mice.

### 2. Topical application of Cutibacterium acnes AD3 strain in the alopecia areata mouse model:

C57BL/6 mice, aged 6-8 weeks, were depilated on the backs using a razor and a depilatory cream, and rested for 8 days. On the 9th day, 3 mg/20 g of cyclophosphamide was intraperitoneally injected to the model mice. After 5 days of observation, hair loss occurred on the head or back of the mice, confirming the successful establishment of the alopecia areata mouse model. The successfully-established model mice were selected for subsequent experiments. Except for the blank group, 100 µl of 1×10⁸ CFU AD3 live bacteria was applied daily on the back for intervention groups. 100 µl of sterile culture medium was applied daily on the back for model group, and 200 µl of 3% minoxidil solution was applied daily on the back for the positive control group. The intervention continued for 7 days, and the hair loss and growth in the mice were regularly observed by photography.

### 3. Topical application of AD3 strain on skin promotes hair regrowth in alopecia areata mouse:

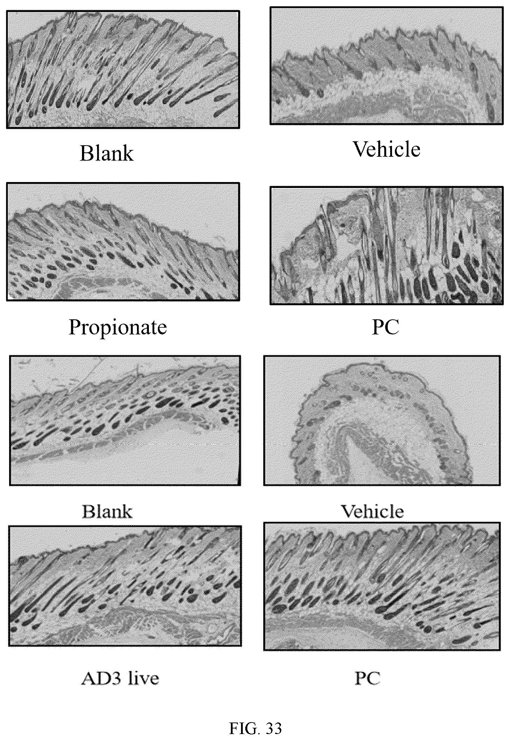

The results are shown in FIGs. 32 and 33. FIG. 32 is the gross photograph of the dorsum of the alopecia areata mouse, wherein, the photos in the first row are photos of treatments in Step 1 for each group, while the photos in the second row are photos of treatments in Step 2 for each group; wherein, Blank: blank group, Vehicle: model group; Propionate: topical propionic acid group; PC: positive control group; AD3 live: topical AD3 live group. FIG. 33 is the HE staining results of skin tissue sections of the alopecia areata mouse. The first and second rows present the staining results of the treatments in Step 1, while the second row shows the staining results from Step 2, wherein, Blank: blank group, Vehicle: model group; Propionate: topical propionic acid group; PC: positive control group; AD3 live: topical AD3 live group. After 9 days of intervention, the hair in the depilated area of the dorsum in the model group was very short and grayish-white colored. The hair in the depilated area of the dorsum in the blank group was longer than that in the model group, with most of the hair being dense, and a few animals exhibited sparse hair, with a gray-black color. The hair in the depilated area of the dorsum in the positive control group are dense, with a deep black color and shiny. The hair in the depilated area of the dorsum in the topical propionic acid group was long, dense, and shiny, with a gray-black color (FIG. 32). The HE staining revealed that the skin in the blank group had a certain number of hair follicles, with melanin formation. The hair follicles in the model group were sparse, with no significant melanin, and in the regression and telogen phase. In both the positive control group and the topical AD3 strain group, there is phenomenon of an increase in the number of hair follicles, active follicular growth, hair papillae being surrounded by hair bulbs, and significant melanin formation (FIG. 33).

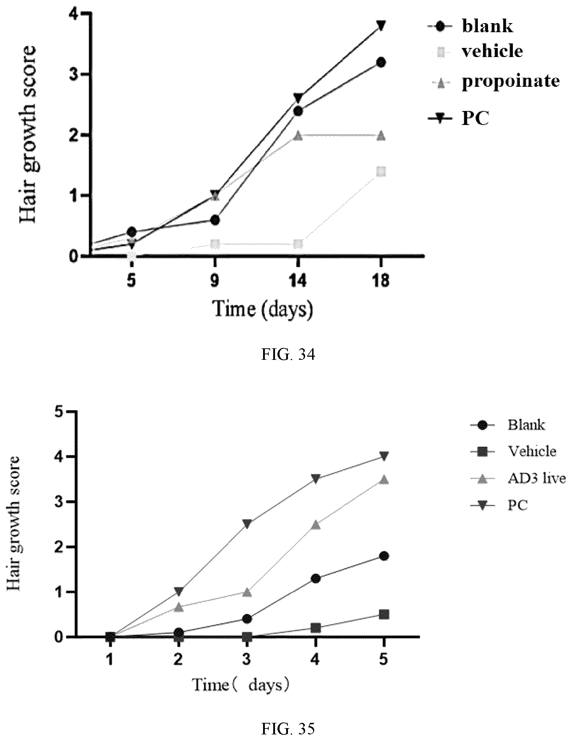

The scoring criteria based on the new hair growth in the depilation area are given as follows: 0 points for pink skin; 1 point for gray skin; 2 points for black skin; 3 points for black skin with some hair growth; 4 points for not showing difference from other areas. Hair growth in different time points for each group of mice was scored. The results are shown in FIGs. 34 and 35. FIG. 34 is the hair growth score differences of alopecia areata mice at different time points for each group in Step 1. FIG. 35 is the hair growth score differences of alopecia areata mice at different time points for each group in Step 2; herein, Blank: blank group; Vehicle: the model group; Propionate: topical propionic acid group; PC: positive control group; AD3 live: topical AD3 live group. As shown in FIGs. 34 and 35, the hair growth in the positive control group increased the fastest over time, followed by the blank group. The hair growth rate of the topical propionic acid group was significantly faster compared to the model group. In the experiment involving topical AD3 live, the hair growth rate of the topical AD3 live group was notably higher than the blank group and the model group, and slightly lower than the positive control group.

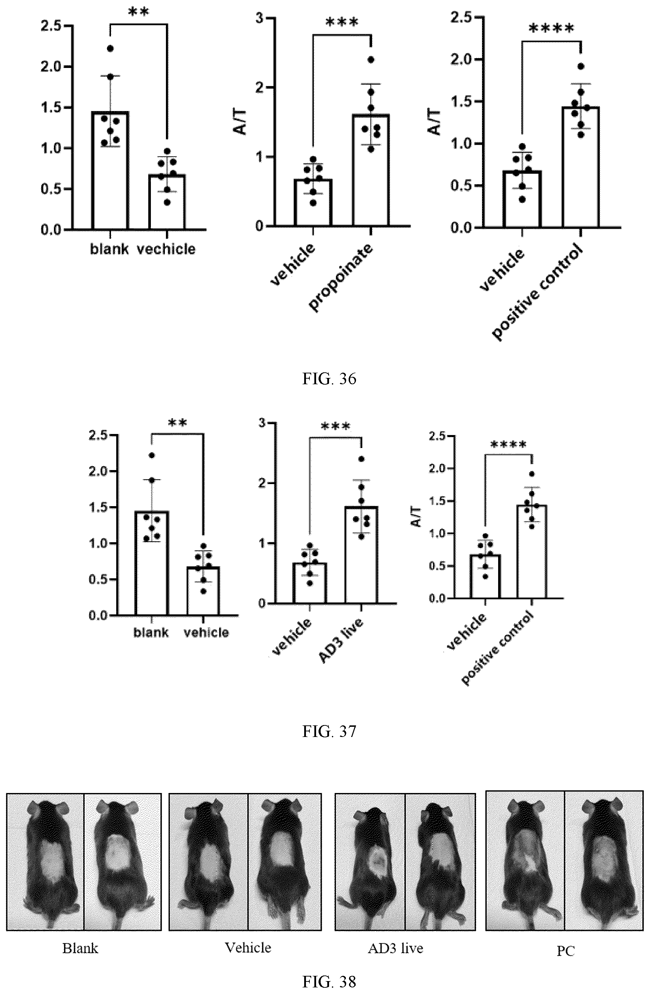

The ratio of follicle number in anagen phase to telogen phase was observed by HE staining for each group. The results are shown in Table 7, Table 8, FIG. 36 and FIG. 37. Table 7 is the statistic results of the ratio of follicle number in anagen phase/telogen phase for each group in Step 1. Table 8 is the statistic results of the ratio of anagen phase/telogen phase for each group in Step 2. FIG. 36 is the ratio of follicle number in anagen phase/telogen phase for each group in Step 1, and FIG. 37 is the ratio of follicle number in anagen phase/telogen phase for each group in Step 2; herein, Blank: blank group; Vehicle: model group; Propionate: topical propionic acid group; PC: positive control group; AD3 live; topical AD3 live group; A: anagen, anagen phase; T: Telogen, telogen phase.

**Table 7: Statistical results of the ratio of follicle number in anagen phase/telogen phase for each group in Step 1**

| | Sample | A/T |
|---|---|---|
| Blank | 7 | 1.45±0.18a |
| Vehicle | 7 | 0.68±0.09 |
| Propionate | 7 | 1.61±0.18a |
| PC | 7 | 1.75±0.13a |

**Table 8: Statistical results of the ratio of follicle number in anagen phase/telogen phase for each group in Step 2**

| | Sample | A/T |
|---|---|---|
| Blank | 7 | 1.23±0.2a |
| Vehicle | 7 | 0.5±0.14 |
| AD3 live | 7 | 1.68±0.09a |
| PC | 7 | 1.72±0.13a |

In Table 7 and Table 8, A:anagen, anagen phase; T: telogen, telogen phase. a: compared with the model group, P < 0.05.

As shown in Table 7, Table 8, FIG. 36, and FIG. 37,
it can be seen that the ratio of follicle number in anagen phase/telogen phase in the model group was significantly lower than that in the blank group (P < 0.05). The ratios of follicle number in anagen phase/telogen phase in the positive control group, the topical AD3 live group and the propionic acid group, were significantly higher compared to the model group (P < 0.01). The differences between the topical AD3 live group and the topical propionic acid group compared to the model group were intermediate between the blank group and the positive control group.

### 4. Topical application of Cutibacterium acnes AD3 in the androgenic alopecia mouse model:

C57BL/6 mice, aged 6 to 8 weeks, were depilated using a razor and a depilatory cream on the back in other groups except the blank group. Testosterone was applied at a dosage of 10 mg·kg⁻¹ on the back of the mice for 21 days. After 21 days, when the back of the mice appeared smooth and pink, without further hair growth, the model was considered successfully established. The successfully established model mice were selected for subsequent experiments. Except for the blank group, 100 µl of 1×10⁸ CFU AD3 live bacteria was applied daily on the back for the intervention groups. 100 µl of sterile culture medium was applied daily on the back for the model group, and 200 µl of 3% minoxidil solution was applied daily on the back for the positive control group. The intervention continued for 10 days, and hair loss and growth in the mice were observed with regular photography.

### 5. Topical application of Cutibacterium acnes AD3 on skin promotes hair regrowth in androgenic alopecia mice:

The results are shown in FIG. 38. FIG. 38 is the gross photograph of the dorsum of androgenic alopecia mice, wherein Blank: blank group; Vehicle: model group; AD3 live: topical AD3 live group; PC: positive control group. As shown in FIG. 38, after 10 days of intervention, the hair in the dorsal depilated area in the model group was very short and grayish-white colored. The hair in the dorsal depilated area in the blank group was gray-black in color than that in the model group. The hair in the dorsal depilated area in the positive control group were dense, deep black colored and shiny. The hair in the dorsal depilated area in the topical AD3 live group was long, dense, and shiny, with a gray-black color.

The above descriptions represent preferred specific embodiments of the present invention. However, the scope of protection of the present invention is not limited to these embodiments. Any skilled person in the field of technology, within the technical scope disclosed by the present invention, may make equivalent substitutions or modifications based on the technical solutions and inventive concepts of the present invention. Such modifications should be considered within the scope of protection of the present invention.

## Claims

1. A *Cutibacterium acnes* (*Cutibacterium acnes* AD3), with a deposit accession number as GDMCC No: 62625.

2. The *Cutibacterium acnes* according to claim 1, wherein the propionic acid produced by every 100,000 CFU of the *Cutibacterium acnes* after 24 hours of cultivation in an anaerobic environment is 200 µg to 1,000 µg.

3. Use of at least one of the *Cutibacterium acnes* according to claim 1 or 2, a cellular component thereof, a metabolite thereof, a derivative of the metabolite thereof, or a secretion thereof in the preparation of at least one of an expression inhibitor of an inflammatory factor, an inhibitor of *Staphylococcus aureus,* an expression promoter of a skin tissue barrier-related molecule, and a neural cell inhibitor.

4. The use according to claim 3, wherein the inflammatory factor comprises at least one of IL-33, IL-1, IL-4, IL-5, IL-6, IL-8, IL-12, IL-13, IL-18, IL-25, IL-31, TSLP, IL-17, IL-22, IL-23, TNF-α, and IFN-γ;
the skin tissue barrier-related molecule comprises at least one of Filaggrin, loricrin, and involucrin.

5. Use of at least one of the *Cutibacterium acnes* according to claim 1 or 2, a cellular component thereof, a metabolite thereof, a derivative of the metabolite thereof, or a secretion thereof in the preparation of a skin-improving product for at least one of treating an inflammation-related skin disease, promoting the balance and homeostasis of skin symbiotic microbiota, promoting skin barrier function, repairing skin damage, or suppressing skin pruritus.

6. The use according to claim 5, wherein the skin-improving product is a medicament or a skincare product.

7. The use according to claim 5, wherein the inflammation-related skin disease comprises at least one of atopic dermatitis, eczema, rosacea, seborrheic dermatitis, contact dermatitis, psoriasis, acne, vitiligo, urticaria, alopecia areata, androgenic alopecia, lupus erythematosus, scleroderma, dermatomyositis, pemphigus, bullous pemphigoid, fungal infectious skin diseases, bacterial infectious skin diseases, viral infectious skin diseases, seborrheic keratosis, actinic keratosis, basal cell carcinoma, and squamous cell carcinoma.

8. A composition, wherein the composition comprises at least one of the *Cutibacterium acnes* according to claim 1 or 2, a cellular component thereof, a metabolite thereof, a derivative of the metabolite thereof, or a secretion thereof, and an excipient.

9. The composition according to claim 8, wherein the excipient comprises at least one of protectants, humectants, emollients, abrasives, salts, or topical formulations.

10. A skin-improving product, wherein the skin-improving product comprises the composition according to claim 8 and a carrier.
